(19) Europäisches Patentamt
European Patent Office
Office européen des brevets

(11) Publication number: 0 025 274 A1

(12) EUROPEAN PATENT APPLICATION

(21) Application number: 80302729.1

(22) Date of filing: 08.08.80

(51) Int. Cl.[3]: C 07 C 143/58
C 07 C 143/63, C 07 C 143/68
C 07 C 143/80, C 07 C 139/00
C 07 D 213/73

(30) Priority: 20.08.79 GB 7928866
30.04.80 GB 8014172

(43) Date of publication of application:
18.03.81 Bulletin 81 11

(84) Designated Contracting States:
BE CH DE FR GB IT LI NL

(71) Applicant: IMPERIAL CHEMICAL INDUSTRIES LIMITED
Imperial Chemical House Millbank
London SW1P 3JF(GB)

(72) Inventor: Bamfield, Peter
18 Links Road Romiley
Stockport(GB)

(72) Inventor: Greenwood, David
66 Sherwood Way Shaw
Oldham, Lancs.(GB)

(74) Representative: Stephenson, Kenneth et al,
Imperial Chemical Industries Limited Legal Department:
Patents Thames House North Millbank
London SW1P 4QG(GB)

(54) **Preparation of aminosulphonic acids and their derivatives.**

(57) A method for the preparation of a compound of the formula:

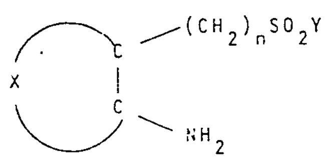

comprising reacting a nitrothiol of the formula:

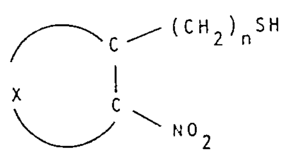

with an active hydrogen compound of the formula HY wherein Y represents the residue of the active hydrogen compound after removal of an active hydrogen atom, X together with the carbon atoms to which it is attached, signifies a carbocyclic or heterocyclic ring system and n is an integer of from 0 to 3. The method is especially suitable for the preparation of orthanilic acid, its amides and esters and their ring substituted analogues.

The products are useful as dyestuff intermediates.

EP 0 025 274 A1

This invention relates to the preparation of aminosulphonic acids and their derivatives.

According to the invention, there is provided a method for the preparation of a compound of the formula:

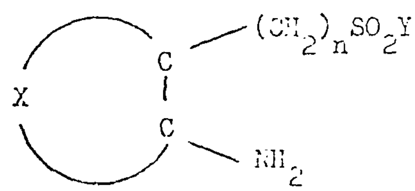

comprising reacting a nitrothiol of the formula:

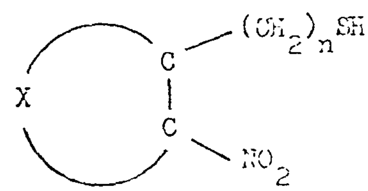

with an active hydrogen compound of the formula HY wherein Y represents the residue of the active hydrogen compound after removal of an active hydrogen atom, X together with the carbon atoms to which it is attached, signifies a carbocyclic or heterocyclic ring system and n is an integer of from 0 to 3.

The active hydrogen compound HY is a compound having an easily replaceable hydrogen atom. Thus, it is a typical nucleophilic reagent and examples of such compounds include water, the product then being a sulphonic acid; an alcohol or phenol, the product then being a sulphonic acid ester; an aliphatic or aromatic thiol, the product then being a thioester; a primary or secondary amine,

the product then being a sulphonic acid amide; a hydrogen halide, the product then being a sulphonic acid halide or an active methylene compound, the product then being a sulphonc.

Preferably, the compound HY is a compound in which Y represents a radical of the formula -OH, $-OR^1$ or $-NR^2R^3$ wherein $R^1$ represents an optionally substituted hydrocarbon radical and each of $R^2$ and $R^3$ represents hydrogen or an optionally substituted hydrocarbon radical. The reaction proceeds particularly smoothly when $R^1$ is an aryl radical such as phenyl and when $R^2$ is hydrogen or alkyl and $R^3$ is an alkyl, cycloalkyl or aryl radical.

The nitrothiol used as starting material may be a carbocyclic or heterocyclic compound and may be of aromatic or non-aromatic character. Examples of heterocyclic compounds include those in which the $-(CH_2)_nSH$ and $-NO_2$ groups are attached to a 5- or 6-membered saturated or unsaturated ring containing one or two hetero atoms, for example nitrogen, oxygen or sulphur. Particularly important carbocyclic nitrothiols are those of the benzene series. Other substituents may be present in the rings of either the carbocyclic or heterocyclic compounds in addition to the $-(CH_2)_nSH$ and $-NO_2$ groups.

The method of the invention is particularly useful for the preparation of aniline-2-sulphonic acid (orthanilic acid) and the amides and esters thereof and their ring substituted analogues from 2-nitrothiophenol and its ring substituted analogues. Examples of substituents which may be present in the benzene ring include halogen atoms, for example chlorine, and lower alkyl, lower alkoxy, nitro, acyl and trifluoromethyl groups.

Other useful starting materials include nitrothiols of the pyridine series and 2-nitrophenylmethanethiol.

The reaction between the nitrothiol and the compound HY is conveniently carried out by heating the two compounds together in appropriate amounts, using a mutual solvent if necessary. Examples of solvents which may be used include lower alkanols, tetrahydrofuran, dioxan and ethyl acetate. The reaction proceeds smoothly at the atmospheric reflux temperatures of the above-mentioned solvents but lower temperatures may be used if desired. The yield of product may be increased by including an acid, for example sulphuric or hydrochloric acid, in the reaction mixture.

After completion of the reaction, the product may be isolated from the solvent by filtration in a very pure form. The products are useful as chemical intermediates, particularly as dyestuff intermediates, especially diazo components for use in the manufacture of azo dyes.

The invention is illustrated but not limited by the following Examples in which all parts and percentages are by weight.

Example 1

2-Nitro-6-chlorothiophenol (1.89 parts) is added to peroxide-free 1,4-dioxan (26 parts) and water (1.2 parts) at room temperature. The solution is heated to reflux temperature (87-88°C) and held there for 10 hours. The precipitated product is then isolated from the hot reaction mixture by filtration, washed with hot dioxan, then with acetone at room temperature and finally dried, giving 1.79 parts of pure 2-amino-6-chlorobenzenesulphonic acid (86.3% yield).

Example 2

2-Nitro-6-chlorothiophenol (1.89 parts) is added to ethanol (20 parts) and 31.5% hydrochloric acid (2.03 parts). The solution is heated to reflux temperature (78-80°C) and maintained for 2½ hours. White crystals are isolated from the hot reaction mixture by filtration, washed with hot ethanol and dried giving 1.5 parts of pure 2-amino-6-chlorobenzenesulphonic acid.

Example 3

2-Nitrothiophenol (1.55 parts) is added to peroxide-free 1,4-dioxan (69 parts) and water (3.5 parts) and the solution is refluxed for 7 hours. The white crystalline product is isolated from the hot reaction medium by filtration, washed with hot dioxan, then with acetone at room temperature and finally dried, giving 1.5 parts of pure 2-aminobenzenesulphonic acid (86.7% yield).

Example 4

2-Nitrothiophenol (1.55 parts) is added to anisole (31 parts) and water (12.8 parts) containing oxyethylated stearic acid (0.1 part). The mixture is refluxed (95-100°C) for 21 hours after which time the aqueous layer is separated and the water removed by evaporation to give 0.94 part of pure 2-aminobenzenesulphonic acid.

The following Table gives further Examples of sulphonic acids which may be prepared from the appropriate nitrothiols using methods analogous to that described in Example 1.

| Example | Starting material | Product |
| --- | --- | --- |
| 5 | 2-nitro-6-methylthiophenol | 2-amino-6-methylbenzene sulphonic acid |
| 6 | 2,6-dinitrothiophenol | 2-amino-6-nitrobenzene sulphonic acid |
| 7 | 2-nitro-6-methoxythio-phenol | 2-amino-6-methoxybenzene sulphonic acid |
| 8 | 2-nitro-4-chlorothiophenol | 2-amino-4-chlorobenzene sulphonic acid |
| 9 | 2-nitro-4-methylthiophenol | 2-amino-4-methylbenzene sulphonic acid |
| 10 | 2,4-dinitrothiophenol | 2-amino-4-nitrobenzene sulphonic acid |
| 11 | 2-nitro-4-trifluoro-methylthiophenol | 2-amino-4-trifluoro-methylbenzene sulphonic acid |
| 12 | 3-nitrobenzophenone-4-thiol | 3-aminobenzophenone-4-sulphonic acid |
| 13 | 2-nitro-5,6-dichloro-thiophenol | 2-amino-5,6-dichloro-benzene sulphonic acid |
| 14 | 3-nitrobenzaldehyde-4-thiol | 3-aminobenzaldehyde-4-sulphonic acid |

Example 15

2-Chloro-5-nitropyridine-6-thiol (1.9 parts) is added to 1,4-dioxan (69 parts) and water (3.5 parts) and the solution is refluxed (87-88°C) for 7 hours. The white crystalline product is isolated by filtration from the hot reaction mixture, washed with dioxan, then acetone and dried giving 5-amino-2-chloropyridine-6-sulphonic acid.

Example 16

2-Nitrothiophenol (3.1 parts) is added to 1,4-dioxan (103 parts) and N-ethylaniline (38 parts). The solution is held at 85-90°C for 18 hours. The presence of 2-aminobenzenesulphon-N-ethylaniline in the reaction mixture is

demonstrated by high performance liquid chromatography. The product is isolated by steam distilling the reaction mixture to remove dioxan and N-ethylaniline giving a partially solid product which, on stirring in water below 10°C gives a pale fawn solid which is isolated by filtration and dried.

Example 17

The procedure of Example 16 is repeated replacing the N-ethylaniline by an equal amount of N-methylcyclohexylamine. The product is 2-aminobenzenesulphon-(N-cyclohexyl-N-methyl)amide.

Example 18

2-Nitrothiophenol (6.2 parts) is added to 1,4-dioxan (206 parts) and phenol (80 parts) and the solution is heated to 85-90°C for 17 hours. The reaction mixture is added to water (1000 parts) maintained alkaline with sodium hydroxide. The crude product (4.6 parts) is filtered off and washed with water. Recrystallisation from ethanol and water (1:1) gives 2-aminobenzene phenyl sulphonate as a pure white solid.

0025274
Dd.70937/cf

CLAIMS

1. A method for the preparation of a compound of the formula:

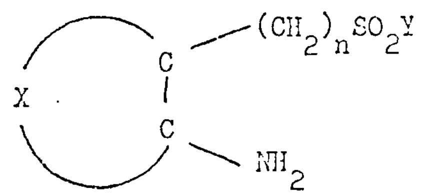

comprising reacting a nitrothiol of the formula:

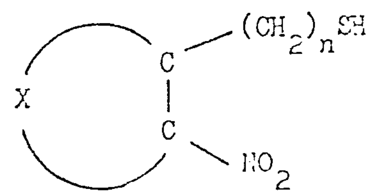

with an active hydrogen compound of the formula HY wherein Y represents the residue of the active hydrogen compound after removal of an active hydrogen atom, X together with the carbon atoms to which it is attached, signifies a carbocyclic or heterocyclic ring system and n is an integer of from 0 to 3.

2. A method according to claim 1 wherein Y represents a radical of the formula -OH, $-OR^1$ or $-NR^2R^3$ wherein $R^1$ represents an optionally substituted hydrocarbon radical and each of $R^2$ and $R^3$ represents hydrogen or an optionally substituted hydrocarbon radical.

3. A method according to claim 1 or claim 2 wherein X together with the carbon atoms to which it is attached, denotes a benzene or pyridine ring.

4. A method according to claim 2 wherein the nitrothiol is 2-nitrothiophenol optionally substituted in the benzene ring by halogen, lower alkyl, lower alkoxy, nitro, acyl or trifluoromethyl.

KS/BH
6.8.80.

European Patent Office

# EUROPEAN SEARCH REPORT

0025274

Application number

EP 80302729.1

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| A | GB - A - 1 517 871 (ICI)<br>+ Page 1, lines 9-86 +<br>-- | 1 | C 07 C 143/58<br>C 07 C 143/63<br>C 07 C 143/68<br>C 07 C 143/80<br>C 07 C 139/00<br>C 07 D 213/73 |
| | GB - A - 11 077 (1898) (DR. FRITZ HAUFF)<br>+ Page 2, line 37 to page 4, line 12 +<br>-- | 1-4 | |
| | DE - A - 2 054 142 (CIBA-GEIGY)<br>+ Pages 1,2,8,9 +<br>-- | 1 | TECHNICAL FIELDS SEARCHED (Int.Cl. 3) |
| | CHEMICAL ABSTRACTS, vol. 85, 1976, Columbus, Ohio, USA<br>V.N. RAJASEKHARAN PILLAI "A new photochemical synthesis of orthanilic acid"<br>pages 436,437, abstract no. 108 375v<br>& Chem. Ind. (London) 1976, (10), 456<br>---- | 1 | C 07 C 143/00<br>C 07 D 213/00<br>C 07 C 139/00 |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 07-11-1980 | REIF |